(19) Europäisches Patentamt
European Patent Office
Office européen des brevets

(11) **EP 3 375 426 B1**

(12) **EUROPEAN PATENT SPECIFICATION**

(45) Date of publication and mention
of the grant of the patent:
**16.10.2019 Bulletin 2019/42**

(51) Int Cl.:
***A61J 1/20*** *(2006.01)*  ***A61J 1/14*** *(2006.01)*
***A61J 1/06*** *(2006.01)*  ***A61J 1/10*** *(2006.01)*
***A61M 5/14*** *(2006.01)*

(21) Application number: **16863697.5**

(22) Date of filing: **13.11.2016**

(86) International application number:
**PCT/CN2016/105563**

(87) International publication number:
**WO 2017/080521 (18.05.2017 Gazette 2017/20)**

(54) **SINGLE-NEEDLE MEDICINE MIXER, DUAL HARD PORTS, AND SOFT INFUSION BAG**

EINZELNADELMEDIKAMENTENMIXER, DUALE HARTE PORTS UND WEICHER INFUSIONSBEUTEL

MÉLANGEUR DE MÉDICAMENT À AIGUILLE UNIQUE, ORIFICES DURS DOUBLES, ET POCHE DE PERFUSION SOUPLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.11.2015 CN 201510780566**

(43) Date of publication of application:
**19.09.2018 Bulletin 2018/38**

(73) Proprietor: **Chongqing Lummy Pharmaceutical Co., Ltd.**
**Chongqing 401123 (CN)**

(72) Inventors:
• **QIU, Yu**
**Chongqing 401123 (CN)**
• **LI, Ke**
**Chongqing 401123 (CN)**

• **ZHANG, Yun**
**Chongqing 401123 (CN)**

(74) Representative: **Huang, Liwei**
**Cäcilienstraße 12**
**40597 Düsseldorf (DE)**

(56) References cited:
**EP-A1- 2 845 577     WO-A1-02/45649**
**CN-A- 102 389 373     CN-A- 102 429 821**
**CN-A- 102 499 889     CN-A- 105 342 850**
**CN-U- 201 987 882     CN-U- 202 061 092**
**CN-U- 202 277 516     CN-U- 202 314 370**
**CN-U- 202 822 124     CN-U- 203 107 704**
**CN-U- 203 123 029     CN-U- 205 339 560**
**JP-A- 2000 070 341     US-A1- 2012 330 267**

## Description

### FILED OF THE INVENTION

The present invention relates to a medication mixer, and in particular to a single-needle medication mixer with a medication mixing cup, dual hard ports and a soft intravenous bag.

### BACKGROUND OF THE INVENTION

In case of using powder injection, freeze-dried powder injection or liquid injection contained in a penicillin bottle, it needs to draw out liquid medication or water for injection in a soft intravenous bag into the penicillin bottle by an injector, until there is enough liquid medication therein; and then to shake the penicillin bottle repeatedly, till the medication in the bottle is mixed evenly. Next, the liquid medication in the penicillin bottle is extracted out into the soft intravenous bag by the injector, until the liquid medication in the bottle is all extracted.

First, the above-mentioned medication mixing process is relatively time-consuming, and strenuous and too many consumable items are spent, such as injectors. More seriously, in the above-mentioned medication mixing process, it is very easy to inject outdoor air into the penicillin bottle and the soft intravenous bag. Under common conditions, there is plenty of various dusts and germs in the air, which may cause very serious medical negligence after the dusts and germs are mixed into the liquid medication to be injected and then into a human body.

Second, as for the traditional infusion preparation, the injection is performed after the medication preparation. The medication in the penicillin bottle is pumped into the soft intravenous bag in advance, and then the medication-prepared soft intravenous bag is brought to an inpatient ward to carry out infusion to a patient. After the medication preparation, the empty penicillin bottle is placed aside. A medical worker has no idea of the medication in the soft intravenous bag, the medication having no traceability. Once the medical worker makes a mistake when preparing the infusion, the consequence is unthinkable.

Third, for some special medications, for example the medication to be used immediately after prepared, the traditional medication preparation is not convenient, and various structures of medication mixers disclosed before the present patent do not solve this problem.

Finally, various structures of medication mixers disclosed before the present invention do not solve the problem of liquid leakage. For the demand on a higher level of medical service, there is an urgent need of an infusion product which is conveniently, safely, and reliably used and has no safety hazard.

In addition, the medication mixer is welded on the soft intravenous bag. After the soft intravenous bag is filled, it needs to perform high temperature sterilization on the whole soft intravenous bag at a temperature of 115-121 degrees Celsius for 30-15 minutes, with a sterilization pressure of 0.15 MPa. Although the medication mixer and the soft intravenous bag are made of polypropylene which can withstand the temperature of 120 degrees Celsius, the material of polypropylene is inevitably softened. Additionally, the high pressure of 0.15 MPa, equivalent to the pressure of 150N per square centimeter, is fatal for the sealed medication mixing cup. First, at a temperature of 120 degrees Celsius, the medication mixing cup and the sealing membrane may have reduced mechanical strength; second, the pressure of 150N per square centimeter directly deforms the body of the medication mixing cup, stretches the sealing membrane, causes wrinkle, and damages the sealing property and the medication mixing cup.

Prior to the present invention, the terminal sterilization of the medication mixer with a sealed medication mixing cup is insurmountable; and the non-sealed medication mixing cup cannot meet the sterility requirement in use. The terminal sterilization of the soft intravenous bag is necessary and obligatory in terms of laws and regulations as well as practical security.

### SUMMARY OF THE INVENTION

The present invention has an object of proposing a soft intravenous bag which is used conveniently and reliably and has no safety hazard, and its related medication mixer and ports.

A medication mixer very much similar to the one of the present invention has been disclosed in EP 2 845 577 which can be regarded as the closest prior art document.

In an aspect of the embodiments according to the present invention, there is provided a single-needle medication mixer, including a base, a medication mixing passage, and a medication mixing cup which are integrated, as well as a piercing needle. The medication mixing cup consists of a cup wall and a cup bottom. The lower end of the medication mixing passage penetrates through the base, and the upper end penetrates through the cup bottom; the upper end of the medication mixing passage is provided with the piercing needle having a hollow passage, the lower end of the piercing needle is connected to the upper end of the medication mixing passage in a sealing way, and the hollow passage is communicated with the medication mixing passage; the medication mixer also comprises a sealing membrane, the sealing membrane being welded at a cup opening of the medication mixing cup in a press welding way, so as to seal the medication mixing cup; and the sealing membrane is an easy-to-tear membrane which can still be basically kept smooth after moist heat terminal sterilization, and the sealing membrane is a breathable easy-to-tear membrane.

Further, a hole communicated with the hollow passage is arranged on a head of a piercing needle, and the piercing needle does not extend out of a cup opening of the medication mixing cup.

Further, the sealing membrane has an air permeability of 5% to 35%.

Further, a metal film is plated on an upper surface of the sealing membrane.

Further, the medication mixer further includes a cover plate; the cover plate is located at an inner edge of the cup opening, and is covered by the sealing membrane.

Further, the cover plate is polygonal, and is provided with a through hole in the center. The head of the piercing needle is located in the through hole, but does not penetrate through the hole; an inner diameter of the through hole is less than an outer diameter of the piercing needle but is greater than the needle point, such that the needle point can extend in the through hole but cannot penetrate therethrough, for supporting the cover plate; reinforcing ribs are arranged on the cover plate.

Further, the medication mixing cup is prefilled with a certain amount of liquid before sealed by the sealing membrane.

Further, the liquid can be vaporized rapidly in an environment of high temperature sterilization.

Further, the gas formed by vaporizing the prefilled certain amount of liquid in the environment of high temperature sterilization may balance the pressure inside and outside the medication mixing cup.

Further, limit protrusions are arranged on an inner wall of the cup wall. After a piercing needle is installed in the medication mixing cup, a needle plate is limited between the limit protrusion and the cup bottom by the limit protrusion, and cannot be taken out.

Further, the limit protrusion is an elastic clamping jaw, the upper ends of which are arranged uniformly and fixed along the periphery of the inner wall of the cup wall, and the lower ends of which are free ends. The elastic clamping jaw inclines towards the center of the medication mixing cup from the fixed upper ends to the free lower ends; the distance of the free end of the elastic clamping jaw to the cup bottom is substantially the same as or slightly greater than the thickness of the penicillin bottle cap, such that after the penicillin bottle is assembled onto the medication mixer, the bottle cap of the penicillin bottle is clamped between the free end and the cup bottom by the elastic clamping jaw.

Further, the limit protrusion is an elastic clamping base which includes a clamping ring which is provided with an elastic clamping jaw at the lower side, the upper ends of the elastic clamping jaw are arranged uniformly and fixed along the clamping ring, and the lower ends of the elastic clamping jaw are free ends. The elastic clamping jaw inclines towards the center of the medication mixing cup from the fixed upper ends to the free lower ends; the distance of the free end of the elastic clamping jaw to the cup bottom is substantially the same as or slightly greater than the thickness of the penicillin bottle cap, such that after the penicillin bottle is assembled onto the medication mixer, the bottle cap of the penicillin bottle is clamped between the free end and the cup bottom by the elastic clamping jaw; the inner wall of the cup wall is provided with a limit structure, and the elastic clamping base is mounted in the medication mixing cup and limited by the limit structure.

Further, the elastic clamping base has a bottom plate, a supporting column, the clamping ring and the elastic clamping jaw which are integrated. The bottom plate is provided with a supporting column at a periphery for supporting and fixing the clamping ring; the bottom plate is provided with a central hole, through which the piercing needle penetrates correspondingly.

Further, the lower end of the medication mixing passage is provided with an easy-breaking handle for sealing the lower end of the medication mixing passage.

According to another aspect of the present invention, there is proposed a medication mixer with a strengthening structure, including any one medication mixer; the cup wall of the medication mixing cup is provided with reinforcing ribs, so as to enhance compressive strength of the cup wall.

Further, the reinforcing ribs are integrally arranged at an inner side of the cup wall in an up and down direction and/or a horizontal direction.

According to another aspect of the present invention, there are also proposed dual hard ports with a medication mixer, including any one medication mixer, one infusion passage arranged at the other side of the base of the medication mixer with respect to the medication mixing passage.

According to another aspect of the present invention, there is provided a soft intravenous bag with dual hard ports, which are the above-mentioned ports.

According to another aspect of the present invention, there is further provided a soft intravenous bag with a medication mixer which is any one medication mixer; the medication mixer is connected with the soft bag by the base.

Further, the medication mixer further includes an infusion port which is arranged on the soft intravenous bag at the same side of the medication mixer, or arranged on the soft intravenous bag at the other side opposite to the medication mixer.

Further, the soft intravenous bag is made of a non-PVC officinal compounding velamen; the base, the medication mixing cup and the membrane which are integrated are made of medical polypropylene, preferably polypropylene R530C; the piercing needle and the limit protrusion are made of polypropylene, preferably polypropylene P17.

BRIEF DESCRIPTION OF THE DRAWINGS

Figure 1 is a schematic diagram of a single-needle medication mixer;

Figure 2 is a local detail diagram of a single-needle head;
Figure 3 is a local detail diagram of a single needle;
Figure 4 is a local detail diagram of a medication mixing cup;
Figure 5 is a schematic diagram of an elastic clamping base;
Figure 6 is a schematic diagram of a cover plate;
Figure 7 is a schematic diagram of a soft intravenous bag with a single-needle medication mixer;
Figure 8 is a schematic diagram of a soft intravenous bag with a single-needle medication mixer in use;
Figure 9 is a schematic diagram of a soft intravenous bag having a single-needle medication mixer with separated infusion and medication mixing; and
Figure 10 is a schematic diagram of a soft intravenous bag having a single-needle medication mixer with separated infusion and medication mixing in use.

Reference numerals:

1, soft intravenous bag, 2, base, 3, medication mixing cup, 3-1, cup wall, 3-2, cup bottom, 3-3, reinforcing rib, 4, limit protrusion/elastic clamping base, 4-1, elastic clamping jaw, 4-2, clamping ring, 4-3, central hole, 4-4, supporting column, 4-5, bottom plate, 5, piercing needle, 5-1, side hole, 5-2, needle point, 5-3, elastic shrink film, 6, easy-breaking handle, 7, medication mixing passage, 8, cover plate, 9, sealing membrane, 10, penicillin bottle, 11, infusion passage, 12, easy-breaking inner cap, 13, rubber plug, 14, easy-breaking outer cap.

DETAILED DESCRIPTION OF THE EMBODIMENTS

In order to make the objectives, technical solution and advantages of the present invention clearer, the present invention is further explained in detail with combination of the embodiments and with reference to the drawings. It shall be understood that the descriptions are only illustrative, but not to limit the scope of the present invention. In addition, in the following explanation, the description of the well-known structure and technology is omitted to avoid unnecessarily confusing the concepts of the present invention.
The present invention will be further explained in combination with drawings of the present invention.

First embodiment

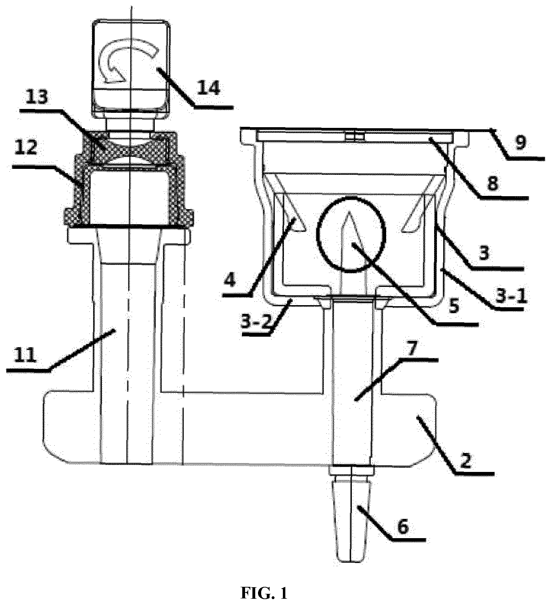

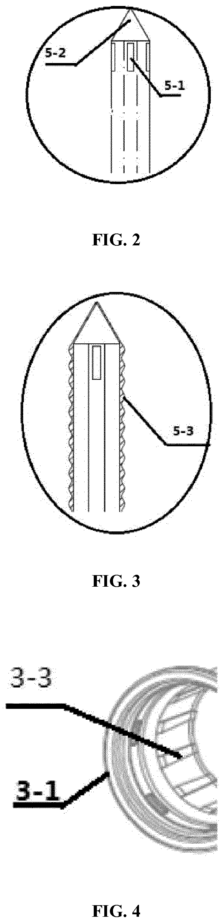

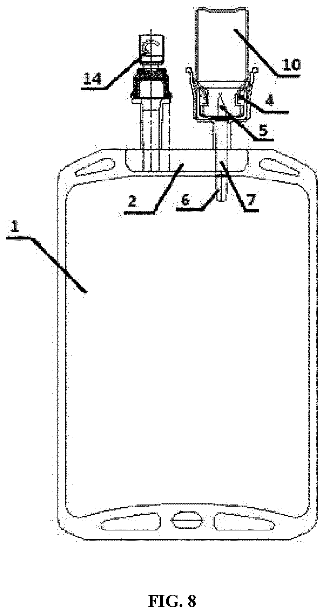

As shown in Figure 1, the medication mixer includes a base 2, a medication mixing cup 3, a limit protrusion 4, a piercing needle 5, a medication mixing passage 7, and an easy-breaking handle 6. The base 2, the medication mixing cup 3 and the medication mixing passage 7 integrally form the body structure of the medication mixer. The easy-breaking handle 6 seals the lower end of the medication mixing passage 7. In the use state as shown in Figure 8, the easy-breaking handle 6 is broken, such that the medication mixing passage 7 is communicated with the penicillin bottle 10 via the piercing needle 5, thereby finishing the medication mixing.
In addition to the medication mixing passage, the medication mixer as shown in Figure 1 may further be integrally provided with an infusion passage 11 arranged on the base 2 parallel to the medication mixing passage 7. The upper end of the infusion passage 11 is provided with an easy-breaking cap, including an inner cap 12, a rubber plug 13 and an easy-breaking cap 14.
The opening of the upper end of the medication mixing cup is provided with a cover plate 8 and a sealing membrane 9 for completely sealing the medication mixing cup.
As for the limit protrusion 4, preferably, the elastic clamping jaw is used; more preferably, the elastic clamping jaw as the limit protrusion 4 is integrated with the medication mixing cup 3.
The upper ends of the elastic clamping jaw 4 are arranged evenly and fixed along the periphery of the inner wall of the cup wall 3-1. The lower ends of the elastic clamping jaw are free ends, and the elastic clamping jaw 4 inclines towards the center of the medication mixing cup from the fixed upper ends to the free lower ends, as shown in Figure 1.
In the medication mixer in use as shown in Figure 8, after the penicillin bottle 10 is assembled and pushed in the medication mixing bottle 3, its bottle cap is pierced by the piercing needle 5 and is clamped by the free end of the elastic clamping jaw 4 and cannot be pulled out. It can be understood that the cap thickness of the penicillin bottle 10 shall be the same as the distance of the free end of the elastic clamping jaw 4 to the cup bottom 3-2 of the medication mixing cup substantially, such that the penicillin bottle 10 is just clamped between the free end of the elastic clamping jaw 4 and the cup bottom 3-2 through the bottle cap, and cannot move up and down.
The piercing needle 5 is arranged at the upper end of the medication mixing passage, and may be detachably connected with the medication mixing passage in a sealing way, preferably, integrated with the body of the medication mixer. As for the needle head of the piercing needle 5, preferably, the needle point 5-2 has a side hole 5-1 as shown in Figure 2. Such an arrangement is to avoid plenty of chippings caused by an edge of an exit of the piercing needle 5 directly cutting a rubber plug or a bottle plug when piercing. By arranging the exit on the side wall of the needle head to form the side hole 5-1 as shown in Figure 2, the needle point 5-2 may be directly pierced into the rubber plug, without direct cutting, which reduces the chippings.
In addition, after the piercing needle 5 pierces the bottle cap, in order to prevent the liquid medication from leaking along the gap between the piercing needle 5 and the penicillin bottle cap, as shown in Figure 3, a layer of elastic shrink film 5-3 is coated on an outer surface of the piercing needle 5, and is compressed after being pierced, stacking around a piercing hole, thereby effectively preventing the liquid medication from leaking out along the gap of the piercing hole.

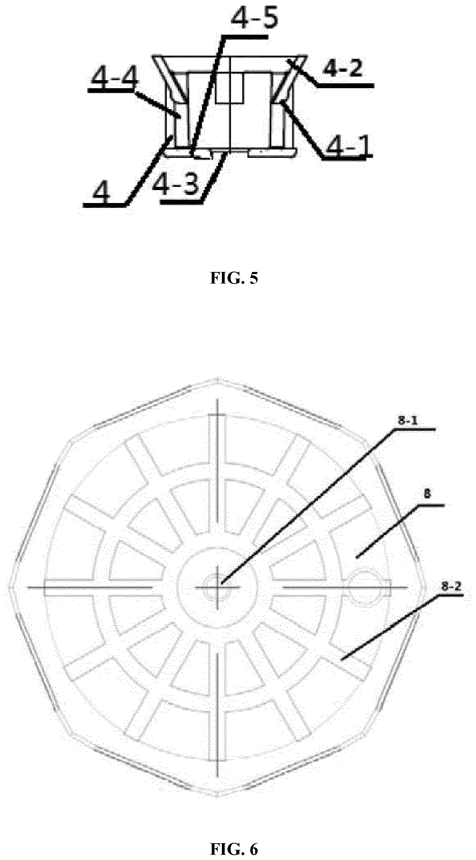

The limit protrusion 4 in the medication mixing cup 3 may be substituted with an independent elastic clamping base 4 as shown in Figure 5, placed in the medication mixing cup 3, for limiting the penicillin bottle.

The elastic clamping base 4 may be designed as follows. As shown in Figure 5, the elastic clamping base 4 includes a clamping ring 4-2 which is provided with an elastic clamping jaw 4-1 at the lower side. The upper ends of the elastic clamping jaw 4-1 are arranged evenly and fixed along the clamping ring 4-2, the lower ends of the elastic clamping jaw 4-1 are free ends, and the elastic clamping jaw inclines towards the center of the medication mixing cup from the fixed upper ends to the free lower ends. The elastic clamping base has a bottom plate 4-5, a supporting column 4-4, a clamping ring 4-2 and an elastic clamping jaw 4-1 which are integrally formed. The supporting column 4-4 is arranged at the periphery of the bottom plate 4-5, for supporting and fixing the clamping ring 4-6.

The bottom plate 4-5 is provided with a central hole 4-3, through which the piercing needle 5 penetrates.

Corresponding to the independent elastic clamping base 4, a limit structure (not shown) is arranged on the inner wall of the cup wall 3-1, and the elastic clamping base 4 is mounted in the medication mixing cup and is limited by the limit structure, such that the elastic clamping base 4 does not slide out of the medication mixing cup after placed. In particular, for the medication mixer in use as shown in Figure 8, after the penicillin bottle 10 is pushed and pierced, the elastic clamping base 4 is limited by the limit structure and cannot withdraw.

With the above-mentioned arrangement, the medication preparation and addition can be performed safely, conveniently and rapidly, which completely solves the problem of secondary pollution at the stage of the medication preparation. Simultaneously, the infusion can be traced since the penicillin bottle 10 cannot be taken out non-destructively after connected and fixed onto the medication mixing cup 3 and clamped by the elastic clamping jaw 4/4-1. That is, from the medication preparation to the completion of infusion, until the recovery, the medication added and injected can be traced.

By using the above-mentioned medication mixer, for the medication needing special preservation, for example, the medication to be used immediately after prepared, the penicillin bottle 10 in which the medication is taken out in a pharmacy is assembled on the medication mixing cup of the soft intravenous bag 1 to be brought to the inpatient ward, and then the easy-breaking handle 6 is broken to finish the medication mixing, thereby realizing that the medication may be used immediately after prepared.

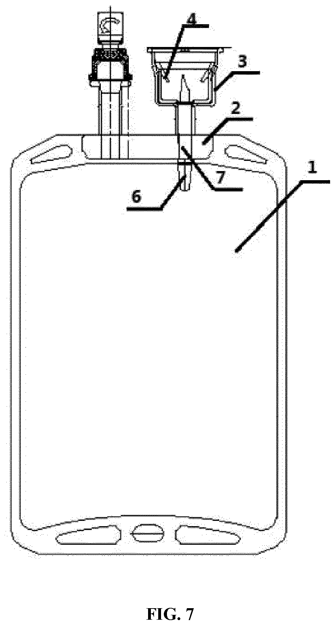

As for the shape and structure of the base 2, in order to avoid the damage of the welding portion of the base to the soft intravenous bag in the processes of storing, transporting and using of the soft intravenous bag with the medication mixer as shown in Figure 7 after the medication mixer, the infusion passage or the dual hard ports with the medication mixer and the infusion passage are welded on the soft bag, the base is designed into a shape of a dumbbell or ship, as shown in Figure 1, and the lower ends of the medication mixing passage 7 and the infusion passage 11 are flushed with the lower end of the base 2. The welding lines are uniformly distributed on the side wall all around the base 2, which may ensure that the base 2 may be well fused and welded with the soft bag 2 even at a relatively low temperature in the welding process of the soft intravenous bag. Moreover, the streamlined base of a shape of ship or dumbbell not only improves mechanical property of welding, but also makes the combination of the dual hard ports with the soft intravenous bag 2 more smooth without a sharp angle, and does not tend to damage the soft bag.

As for the selection of the materials of the medication mixer and the soft intravenous bag, the soft intravenous bag is made of the widely used non-PVC officinal compounding velamen, including three or five layers.

The medication mixer is made of the medical polypropylene material having good compatibility with the non-PVC material of the soft intravenous bag. The base, the medication mixing cup and the cover plate are preferably made of the polypropylene R530C material; however, the piercing needle, the elastic clamping base and the limit structure are preferably made of the P17 material in the pp material system in view of its piercing property and mechanical characteristics.

Finally, the most critical point of the medication mixer with a sealing structure is the terminal sterilization after the sealed medication mixer is welded on the soft bag. Currently, from the point of view of laws and regulations as well as practical injection safety, the terminal sterilization must be performed to all the medication packages before the filling and delivery.

At present, there are mainly two sterility assurance processes for the injection:

    1. A process of terminal sterilization: on the basis of controlling a pollution load of microorganism, after the medication is filled, the degerming is realized by moist heat sterilization. Usually, this method has a low cost, a high level of sterility assurance, and is suitable for sterilizing both a large volume injection and a small volume injection.

    2. A process of sterile production: under an environment of a sterile system, by sterile filtration or sterile operation, for the purpose of de-pollution, the sterility level is assured by eliminating various possibilities of causing pollutions. Generally, due to a high demand of this method on the environment system, and many factors of influencing the sterile operation, the sterility assurance level is lower than that in the terminal sterilization process. The sterile production process is usually suitable for powder-injection, and also for clinical needs, but not for the small volume injector which may be realized in the terminal sterilization. Thus, the terminal sterilization process has different system requirements, different sterilization methods and different sterilization assurance results from the sterile production process.

The large volume infusion is very sensitive to the cost. Therefore, the terminal sterilization in the large volume infusion may only adopt the moist heat sterilization process with a low cost and high efficiency, which is usually conducted at a high temperature of 115-121 degrees Celsius, under the steam with a pressure of 0.15 MPa, for 30-15 minutes.

Although the medication mixer and the soft intravenous bag are made of the polypropylene material withstanding the high temperature of 120 degrees Celsius, at such a high temperature, the sealed medication mixer will degrade in mechanical characteristics, and tends to deform under the intensity of pressure of 0.15 MPa; however, the sealing membrane will also be deformed and wrinkled at this temperature and intensity of pressure, losing the sealing effect.

From the year 2011 to 2014, hundreds of experiments were conducted, so as to solve the problem of terminal sterilization of the sealed medication mixer and the soft intravenous bag.

First, as for the structure of the medication mixing cup, as shown in Figure 4, the reinforcing ribs 3-3 are arranged at the lower half portion of the cup body. The reinforcing ribs are arranged at the cup wall of the medication mixing cup, preferably, at the lower half portion of the cup wall.

The reinforcing ribs may be protruding vertical bars or horizontal bars which are integrated with the medication mixing cup and uniformly arranged at the inner side and/or outer side of the cup wall, or may have a criss-crossed net structure. Preferably, the vertical bars are uniformly arranged at the lower half portion of the inner side of the cup wall; more preferably, the protruding vertical bars extend to the cup bottom from the lower half portion of the inner side of the cup wall, and most preferably, the thickness of the vertical protrusion continuously increases gradually and smoothly from top to bottom.

The medication mixing cup with the reinforcing ribs improves the mechanical pressure resistance of the cup body to a considerable extent. With the experimental comparison, the medication mixing cup without the reinforcing ribs is compressed into a square from the initial circular shape after the moist heat sterilization is performed, and cannot be used any more.

After the reinforcing ribs are arranged, subsequent to the moist heat sterilization process, the circular medication mixing cup body is slightly compressed, and can be continue to use normally.

Moreover, the sealing membrane 9 is only a very thin easy-to-tear film, for sealing the medication mixing cup 3 and being convenient to tear out in use. The sealing membrane 9 is thinner than the cup body of the medication mixing cup 3 since the sealing membrane 9 itself is only a layer of thin film with a thickness of micron dimension, and cannot withstand the intensity of pressure of 0.15 MPa in the moist heat sterilization process.

As for this, numerous experiments show that the cover plate 8 as shown in Figure 6 is arranged under the sealing membrane 9, and is covered by the sealing membrane 9, such that the cover plate 8 effectively supports the sealing membrane 9, so as to prevent the sealing membrane 9 from being compressed and deformed in sterilization. The cover plate 8 is placed on an inner stepwise edge of the cup opening of the medication mixing cup, such that the upper surface of the cover plate arranged at the cup opening is flushed with or slightly lower than the cup opening. Such an arrangement of the cover plate 8 does not influence the sealing process of press welding the sealing membrane 9 on the upper end surface of the cup wall.

The cover plate has a circular shape substantially matched with the shape of the cup opening of the medication mixing cup. Preferably, the cover plate 8 has a shape in cross section of polygon, for example, pentagon, hexagon, octagon and dodecagon. The polygonal cover plate 8 is conveniently taken out in use; its another unexpected effect will be mentioned in the following.

In addition, in order to enhance the compressive strength of the cover plate, preferably, the cover plate 8 is arranged coaxially with the piercing needle, with its point 5-2 dead against the center of the cover plate, for supporting the cover plate. More preferably, one through hole 8-1 is arranged in the center of the cover plate 8 which has an inner diameter less than an outer diameter of the piercing needle 5. The needle point 5-2 is partially located in the through hole, but cannot penetrate therethrough. That is, the needle point 5-2 is embedded in the through hole 8-1 of the cover plate 8, thereby better supporting the cover plate 8 by the piercing needle 5.

Simultaneously, in view of an intense downward pressure born by the cover plate 8, the cover plate 8 is reinforced other than the design of supporting the cover plate 8 by the piercing needle 5. The radial and annular reinforce ribs 8-2 as shown in Figure 6 enhance the mechanical strength of the cover plate 8.

The sealing membrane 9 press welded on the upper end of the cup opening is tightly stuck on the upper surface of the cover plate 9, such that the sealing membrane 9 does not need to withstand a high pressure, which greatly buffer the deformation and wrinkle of the sealing membrane 9.

As for the wrinkle, since the sealing membrane 9 is too thin, the wrinkle will be caused even by a slight pressure, which seriously influences the vision effect of the product.

By many contrast experiments, a layer of thin metal film is plated on the upper surface of the sealing membrane 9, for example, an aluminized sealing membrane may effectively alleviate the problem of wrinkle of the sealing membrane.

Although the sealed medication mixer with such an arrangement withstands the high temperature and high pressure in the moist heat sterilization process and can be used normally, the product appearance cannot be kept in a good state. In view of the infusion product, it is not qualified.

The above-mentioned design for the structure of the medication mixer can only ensure the use function. In order to completely solve the problem of terminal sterilization of the sealed medication mixer, it needs to fundamentally solve the balance of air pressure inside and outside in the moist heat sterilization of the sealed medication mixing cup.

After the medication mixing cup is assembled and before the cover plate 8 and the sealing membrane 9 are mounted to seal the medication mixing cup, a certain amount of liquid is prefilled in the cup body, and then the cover plate 12 mounted to seal the medication mixing cup.

When the terminal sterilization is performed on the sealed medication mixing cup with liquid filled in and the soft intravenous bag, the liquid is rapidly vaporized at a high temperature, thereby balancing the pressure inside and outside the cup body rapidly.

As for the prefilled liquid, preferably, the thermal capacity is relatively small, to saturate the liquid with a relatively high steam pressure. We have studied that the states of various liquid at a temperature of 120 degrees Celsius under the pressure of 0.15MPa, including common harmless liquid such as water and ethyl alcohol, can meet our requirements. In view of costs and safety, preferably, the prefilled liquid is water.

The important prefilled water amount $V_0$ can be confirmed through the following equation:

$$PV = nRT,$$

wherein P is an inside-outside pressure difference in the moist heat sterilization, V is a volume of the medication mixing cup, n is a mole number of the prefilled liquid/water, R is a gas constant, and T is an absolute temperature in the moist heat sterilization.

$$V_0 = n*M/\rho,$$

wherein M is a mole mass of the liquid/water, and $\rho$ is a density of the liquid/water.

According to the above-mentioned equation, the pressure inside and outside in the terminal sterilization of the sealed medication mixer can be well balanced.

It is a preferable solution that the pressure inside and outside is balanced in a manner of prefilled liquid.

However, the above-mentioned solution may solve the problem of pressure balance. In practical use, there still exist some problems. The most typical problem is that the finished soft intravenous bag subjected to sterilization, after being cooled, has water drops or liquid in the medication mixing cup, which affects the impression. Moreover, such a product is not accepted by hospitals or patients.

On this basis, one more preferably solution is that we have specially studied the breathable sealing membrane based on the pp material system, which ensures sufficient gas exchange of the breathable sealing membrane with outside after the vaporization of liquid, so there is no residual liquid in the medication mixing cup after the sterilization.

Through many experiments for a long time, the air permeability of the breathable sealing membrane is, most preferably, 5%-35%.

As for the medication mixer sealed by our breathable sealing membrane, subsequent to the moist heat high temperature sterilization, the medication mixing cup is not deformed, and the sealing membrane is smooth as before, without any wrinkle.

Of course, one improvement solution is that the medication mixer is sealed by the breathable sealing membrane with a suitable permeability without pre-filling liquid. Through many experiments, the sealing membrane with the air permeability ranging between 25% and 35%, preferably, 30% may basically ensure the integrity of the structure of the medication mixer after the sterilization.

The cover plate is preferably designed into a polygon with a hole in the center. The experiment shows that after the pressure balancing means is adopted, such a preferable design can balance the pressure inside and outside the medication mixing cup more rapidly, thereby functioning very well in the moist heat sterilization process---the sealing membrane is smooth as before, and the medication mixing cup is not deformed. This is because the polygonal cover plate with a central hole can increase the gas exchanging speed below and above the cover plate, and balance the air pressure in the medication mixing cup more rapidly, as well as the air pressure outside and inside the medication mixing cup.

Second embodiment

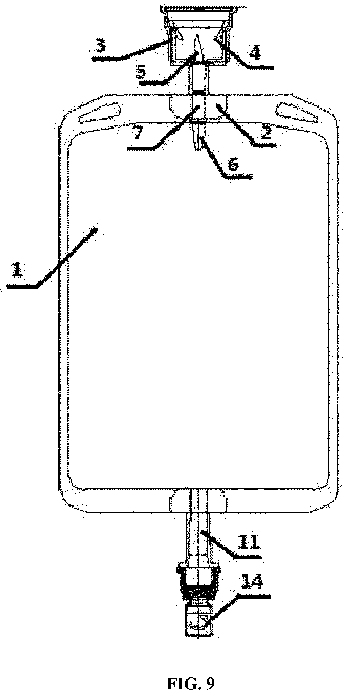

As shown in Figure 9, the medication mixing cup 3 and the infusion passage 11 are arranged separately, and are located at the upper and lower sides of the soft intravenous bag. Of course, it can be understood that the medication mixer and the infusion passage are separately arranged at the same side of the soft intravenous bag. The medication mixer, the infusion passage and the ports in the present embodiment are substantially the same as those in the first embodiment, with the difference that the medication mixer and the infusion passage 11 as well as the ports consisting of the inner cover 12, the easy-breaking outer cover 14 and the rubber plug in the present embodiment are separately arranged.

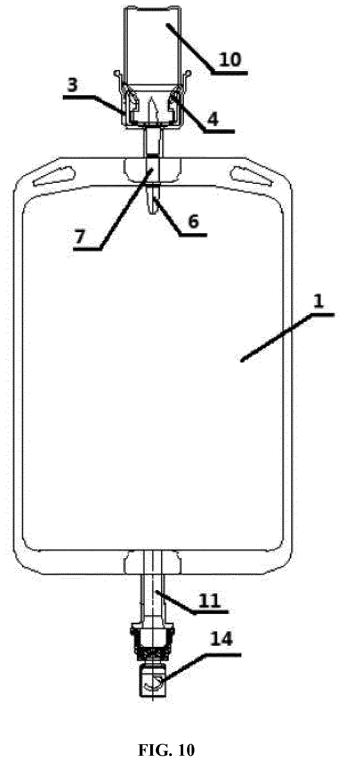

Figure 10 shows the soft intravenous bag with the medication mixer in use. The penicillin bottle 10 is assembled and pushed in the medication mixing cup 3, with the bottle cap pierced by the piercing needle 5, while the penicillin bottle 10 is clamped by the elastic clamping jaw 4 at the bottle neck under the bottle cap, and cannot withdraw non-destructively.

It shall be understood that the above embodiments of the present invention are only used for illustratively explaining the principle of the present invention, without limiting the present invention.

## Claims

1. A single-needle medication mixer, comprising a base (2), a medication mixing passage (7), a medication mixing cup (3) and a piercing needle (5), wherein the base (2), the medication mixing passage (7) and the medication mixing cup (3) are integrated, and the medication mixing cup (3) consists of a cup wall (3-1) and a cup bottom (3-2), wherein the lower end of the medication mixing passage (7) penetrates through the base (2) and the upper end penetrates through the cup bottom (3-2); the upper end of the medication mixing passage (7) is provided with the piercing needle (5) having a hollow passage (7), the lower end of the piercing needle (5) is connected to the upper end of the medication mixing passage (7) in a sealing way, and the hollow passage (7) is communicated with the medication mixing passage (7); the medication mixer also comprises a sealing membrane (9), the sealing membrane (9) being welded at a cup opening of the medication mixing cup (3) in a press welding way, so as to seal the medication mixing cup (3); the sealing membrane (9) is an easy-to-tear membrane which can still be basically kept smooth after moist heat terminal sterilization, and the sealing membrane (9) is a breathable easy-to-tear membrane.

2. The medication mixer according to claim 1, wherein the sealing membrane has an air permeability of 5% to 35%.

3. The medication mixer according to claim 1, wherein a metal film is plated on an upper surface of the sealing membrane.

4. The medication mixer according to claims 1-3, further comprising a cover plate; the cover plate is located at an inner edge of the cup opening, and is covered by the sealing membrane.

5. The medication mixer according to claim 1, wherein the cover plate is polygonal, and is provided with a through hole in the center, the head of the piercing needle is located in the through hole, but does not penetrate through the hole; an inner diameter of the through hole is less than an outer diameter of the piercing needle but is greater than the needle point, such that the needle point can extend in the through hole but cannot penetrate therethrough, for supporting the cover plate; reinforcing ribs are arranged on the cover plate.

6. The medication mixer according to claims 1-5, wherein the medication mixing cup is prefilled with a certain amount of liquid before sealed by the sealing membrane; the liquid can be vaporized rapidly in an environment of high temperature sterilization; the gas formed by vaporizing the pre-filled certain amount of liquid in the environment of high temperature sterilization may balance the pressure inside and outside the medication mixing cup.

7. The medication mixer according to any one of claims 1-6, wherein limit protrusions are arranged on an inner wall of the cup wall; after a piercing needle is installed in the medication mixing cup, a needle plate is limited between the limit protrusion and the cup bottom by the limit protrusion, and cannot be taken out.

8. The medication mixer according to claim 7, wherein the limit protrusion is an elastic clamping jaw, the upper ends of which are arranged uniformly and fixed along the periphery of the inner wall of the cup wall, and the lower ends of which are free ends, and the elastic clamping jaw inclines towards the center of the medication mixing cup from the fixed upper ends to the free lower ends; the distance of the free end of the elastic clamping jaw to the cup bottom is substantially the same as or slightly greater than the thickness of the penicillin bottle cap, such that after the penicillin bottle is assembled onto the medication mixer, the bottle cap of the penicillin bottle is clamped between the free end and the cup bottom by the elastic clamping jaw.

9. The medication mixer according to claim 7, wherein the limit protrusion is an elastic clamping base which includes a clamping ring which is provided with an elastic clamping jaw at the lower side, the upper ends of the elastic clamping jaw are arranged uniformly and fixed along the clamping ring, and the lower ends of the elastic clamping jaw are free ends, and the elastic clamping jaw inclines towards the center of the medication mixing cup from the fixed upper ends to the free lower ends; the distance of the free end of the elastic clamping jaw to the cup bottom is substantially the same as or slightly greater than the thickness of the penicillin bottle cap, such that after the penicillin bottle is assembled onto the medication mixer, the bottle cap of the penicillin bottle is clamped between the free end and the cup bottom by the elastic clamping jaw; the inner wall of the cup wall is provided with a limit structure, and the elastic clamping base is mounted in the medication mixing cup and limited by the limit structure.

10. The medication mixer according to claim 7, wherein the elastic clamping base has a bottom plate, a supporting column, the clamping ring and the elastic clamping jaw which are integrated, the bottom plate is provided with a supporting column at a periphery for supporting and fixing the clamping ring; the bottom plate is provided with a central hole, through which the piercing needle penetrates correspondingly.

11. The medication mixer according to any one of claims 1-10, wherein the lower end of the medication mixing passage is provided with an easy-breaking handle for sealing the lower end of the medication mixing passage.

12. A medication mixer with a strengthening structure, comprising any one medication mixer according to claims 1-11; the cup wall of the medication mixing cup is provided with reinforcing ribs, so as to enhance compressive strength of the cup wall; the reinforcing ribs are integrally arranged at an inner side of the cup wall in an up and down direction and/or a horizontal direction.

13. Dual hard ports with a medication mixer, comprising any one medication mixer according to claims 1-12, wherein one infusion passage is arranged at the other side of the base of the medication mixer with respect to the medication mixing passage.

14. A soft intravenous bag with dual hard ports, wherein the dual hard ports are the ones according to claim 13.

15. A soft intravenous bag with a medication mixer, comprising a medication mixer and a soft intravenous bag, the medication mixer is the one according to any one of claims 1-12; the medication mixer is connected with the soft bag by the base; further comprising an infusion port which is arranged on the soft intravenous bag at the same side of the medication mixer, or arranged on the soft intravenous bag at the other side opposite to the medication mixer.

**Patentansprüche**

1. Einnadel-Medikamentenmischer, umfassend eine Basis (2), einen Medikamentenmischkanal (7), einen Medikamentenmischbecher (3) und eine Stechnadel (5), wobei die Basis (2), der Medikamentenmischkanal (7) und der Medikamentenmischbecher (3) integriert sind, und wobei der Medikamentenmischbecher (3) eine Becherwand (3-1) und einen Becherboden (3-2) aufweist, und wobei das untere Ende des Medikamentenmischkanal (7) die Basis (2) durchdringt und das obere Ende den Becherboden (3-2) durchdringt;
und wobei das obere Ende des Medikamentenmischkanals (7) mit der Stechnadel (5) versehen ist, die einen hohlen Kanal (7) aufweist, und wobei das untere Ende der Stechnadel (5) auf eine abdichtende Weise mit dem oberen Ende des Medikamentenmischkanals (7) verbunden ist, und wobei der hohle Kanal (7) ist mit dem Medikamentenmischkanal (7) verbunden ist;
und wobei der Medikamentenmischer auch eine Dichtungsmembran (9) umfasst, und wobei die Dichtungsmembran (9) an einer Becheröffnung des Medikamentenmischbechers (3) auf eine Pressschweißweise angeschweißt ist, um den Medikamentenmischbecher (3) abzudichten;
und wobei die Dichtungsmembran (9) eine leicht zu zerreißende Membran ist, die nach einer Sterilisation an einem feuchten Hitzeterminal im Wesentlichen noch glatt gehalten werden kann, und wobei die Dichtungsmembran (9) eine atmungsaktive, leicht zu zerreißende Membran ist.

2. Medikamentenmischer nach Anspruch 1, wobei die Dichtungsmembran eine Luftdurchlässigkeit von 5% bis 35% aufweist.

3. Medikamentenmischer nach Anspruch 1, wobei ein Metallfilm auf einer oberen Oberfläche der Dichtungsmembran plattiert ist.

4. Medikamentenmischer nach Anspruch 1-3, wobei er weiterhin eine Abdeckplatte umfasst;
und wobei die Abdeckplatte sich an einem inneren Rand der Becheröffnung befindet und durch die Dichtungsmembran abgedeckt wird.

5. Medikamentenmischer nach Anspruch 1, wobei die Abdeckplatte polygonal ausgebildet und in der Mitte mit einem Durchgangsloch versehen ist, und wobei der Kopf der Stechnadel sich im Durchgangsloch befindet, aber das Loch nicht durchdringt;
Und wobei ein Innendurchmesser des Durchgangslochs kleiner als ein Außendurchmesser der Stechnadel, allerdings größer als die Nadelspitze ist, so dass sich die Nadelspitze in dem Durchgangsloch erstrecken, aber dort nicht durchdringen kann, um die Abdeckplatte zu stützen;
Und wobei auf der Abdeckplatte Verstärkungsrippen angeordnet sind.

6. Medikamentenmischer nach den Ansprüchen 1-5, wobei der Medikamentenmischbecher vor dem Abdichten durch die Dichtungsmembran mit einer bestimmten Menge an Flüssigkeit vorgefüllt wird;
und wobei die Flüssigkeit in einer Umgebung der Hochtemperatursterilisation schnell verdampft werden kann;
und wobei das durch Verdampfen der vorgefüllten Menge an Flüssigkeit in der Umgebung der Hochtemperatursterilisation gebildete Gas den Druck innerhalb und außerhalb des Medikamentenmischbechers ausgleichen kann.

7. Medikamentenmischer nach einem der Ansprüche 1-6, wobei Begrenzungsvorsprünge an einer Innenwand der Becherwand angeordnet sind; und wobei eine Stechplatte zwischen dem Begrenzungsvorsprung und dem Becherboden durch den Begrenzungsvorsprung begrenzt ist und nicht herausgenommen werden kann, nachdem eine Stechnadel in dem Medikamentenmischbecher installiert wurde.

8. Medikamentenmischer nach Anspruch 7, wobei es sich bei dem Begrenzungsvorsprung um eine elastische Klemmbacke handelt, deren oberen Enden gleichmäßig angeordnet und entlang des Umfangs der Innenwand der Schalenwand befestigt sind und deren unteren Enden freie Enden sind, und wobei die elastische Klemmbacke sich zur Mitte des Medikamentenmischbechers von den festen oberen Enden zu den freien unteren Enden neigt;
und wobei der Abstand des freien Endes der elastischen Klemmbacke zum Becherboden im Wesentlichen gleich wie oder geringfügig größer als die Dicke des Penicillinflaschenverschlusses ist, so dass nach dem Anbringen der Penicillinflasche auf den Medikamentenmischer der Flaschenverschluss der Penicillinflasche durch die elastische Klemmbacke zwischen dem freien Ende und dem Becherboden eingeklemmt ist.

9. Medikamentenmischer nach Anspruch 7, wobei es sich bei dem Begrenzungsvorsprung um eine elastische Klemmbasis handelt, die einen Klemmring umfasst, und wobei der Klemmring an der Unterseite mit einer elastischen Klemmbacke versehen ist, und wobei die oberen enden der elastischen Klemmbacke gleichmäßig angeordnet und entlang dem Klemmring befestigt sind, während die unteren Enden der Klemmbacke freie Enden sind, und wobei die elastische Klemmbacke sich zur Mitte des Medikamentenmischbechers von den festen oberen Enden zu den freien unteren Enden neigt;
und wobei der Abstand des freien Endes der elastischen Klemmbacke zum Becherboden im Wesentlichen gleich wie oder geringfügig größer als die Dicke des Penicillinflaschenverschlusses ist, so dass nach dem Anbringen der Penicillinflasche auf den Medikamentenmischer der Flaschenverschluss der Penicillinflasche durch die elastische Klemmbacke zwischen dem freien Ende und dem Becherboden eingeklemmt ist.
und wobei die Innenwand der Becherwand mit einer Begrenzungsstruktur versehen ist, und wobei die elastische Klemmbasis in dem Medikamentenmischbecher angebracht und durch die Begrenzungsstruktur begrenzt ist.

10. Medikamentenmischer nach Anspruch 7, wobei die elastische Klemmbasis eine Bodenplatte, eine Tragsäule, den Klemmring und die elastische Spannbacke aufweist, die integriert sind, und wobei die Bodenplatte an einer Peripherie mit einer Tragsäule zum Unterstützen und Befestigen des Klemmrings versehen ist;
und wobei die Bodenplatte mit einer zentralen Bohrung versehen ist, durch die die Stechnadel entsprechend durchdringt.

11. Medikamentenmischer nach einem der Ansprüche 1-10, wobei das untere Ende des Medikamentenmischkanals mit einem leicht zu brechenden Griff versehen ist, um das untere Ende des Medikamentenmischkanals abzudichten.

12. Medikamentenmischer mit einer Verstärkungsstruktur, umfassend einen Medikamentenmischer nach den Ansprüchen 1 bis 11;
wobei die Becherwand des Medikamentenmischbechers mit Verstärkungsrippen versehen ist, um die Druckfestigkeit der Becherwand zu verbessern;
und wobei die Verstärkungsrippen einteilig an einer Innenseite der Becherwand in einer Aufwärts- und Abwärtsrichtung und/oder einer Horizontalrichtung angeordnet sind.

13. Dual-Hart-Anschlüsse mit einem Medikamentenmischer, umfassend einen Medikamentenmischer nach den Ansprüchen 1-12, wobei ein Infusionskanal an der anderen Seite der Basis des Medikamentenmischers in Bezug auf den Medikamentenmischkanal angeordnet ist.

14. Weicher intravenöser Beutel mit Dual-Hart-Anschlüsse, wobei die Dual-Hart-Anschlüsse diejenigen gemäß dem Anspruch 13 sind.

15. Weicher intravenöser Beutel mit einem Medikamentenmischer, umfassend einen Medikamentenmischer und einen weichen intravenösen Beutel, wobei der Medikamentenmischer der nach einem der Ansprüche 1-12 ist;

und wobei der Medikamentenmischer über die Basis mit dem weichen Beutel verbunden ist;

und wobei der Beutel weiterhin eine Infusionsöffnung umfasst, die an dem weichen intravenösen Beutel auf der gleichen Seite des Medikamentenmischers oder an dem weichen intravenösen Beutel auf der anderen Seite gegenüber dem Medikamentenmischer angeordnet ist.

## Revendications

1. Mélangeur de médicaments à une aiguille, comprenant un passage de mélange de médicaments (7), une tasse de mélange de médicaments (3) et une aiguille de perforation (5), la base (2), le passage de mélange de médicaments (7) et la tasse de mélange de médicaments (3) sont intégrés, et la tasse de mélange de médicaments (3) se compose d'une paroi de tasse (3-1) et d'un fond de tasse (3-2), dans lequel : l'extrémité inférieure du passage de mélange de médicaments (7) pénètre à travers la base (2) et l'extrémité supérieure pénètre à travers le fond de la tasse (3-2) ;

l'extrémité supérieure du passage de mélange de médicaments (7) est munie d'une aiguille de perforation (5) comportant un passage creux (7), l'extrémité inférieure de l'aiguille de perforation (5) est reliée à l'extrémité supérieure du passage de mélange de médicaments (7) de manière étanche, et le passage creux (7) est en communication avec le passage de mélange de médicaments (7) ;

le mélangeur de médicaments comprend également une membrane d'étanchéité (9), la membrane d'étanchéité (9) étant soudée au niveau d'une ouverture de la tasse de mélange de médicaments (3) d'une manière à souder par pression, de manière à sceller la tasse de mélange de médicaments (3) ;

la membrane d'étanchéité (9) est une membrane facile à déchirer qui peut encore rester fondamentalement lisse après la stérilisation par chauffage à la chaleur humide, et la membrane d'étanchéité (9) est une membrane respirante facile à déchirer.

2. Mélangeur de médicaments selon la revendication 1, dans lequel la membrane d'étanchéité a une perméabilité à l'air de 5% à 35%.

3. Mélangeur de médicaments selon la revendication 1, dans lequel un film métallique est plaqué sur une surface supérieure de la membrane d'étanchéité.

4. Mélangeur de médicaments selon les revendications 1 à 3, comprenant en outre une plaque de couverture ;

la plaque de couverture est située sur un bord intérieur de l'ouverture de la tasse et est recouverte par la membrane d'étanchéité.

5. Mélangeur de médicaments selon la revendication 1, dans lequel la plaque de couverture est polygonale et est pourvue d'un trou traversant au centre, la tête de l'aiguille de perforation est située dans le trou traversant, mais ne pénètre pas à travers le trou ;

un diamètre intérieur du trou traversant est inférieur au diamètre extérieur de l'aiguille de perforation mais est supérieur à la pointe de l'aiguille, de telle sorte que la pointe de l'aiguille puisse s'étendre dans le trou traversant mais ne peut pas y pénétrer pour supporter la plaque de couverture ;

des nervures de renforcement sont disposées sur la plaque de couverture.

6. Mélangeur de médicament selon les revendications 1 à 5, dans lequel la tasse de mélange de médicaments est pré-remplie d'une certaine quantité de liquide avant d'être scellé par la membrane d'étanchéité ;

le liquide peut être vaporisé rapidement dans un environnement de stérilisation à haute température ;

le gaz formé en vaporisant une certaine quantité de liquide pré-remplie dans l'environnement de stérilisation à haute température peut équilibrer la pression à l'intérieur et à l'extérieur de la tasse de mélange de médicaments.

7. Mélangeur de médicaments selon l'une quelconque des revendications 1 à 6, dans lequel des saillies limites sont disposées sur une paroi interne de la paroi de la tasse ;

une fois l'aiguille perforante installée dans la tasse de mélange de médicaments, la plaque de l'aiguille est limitée entre la saillie limite et le fond de tasse par la saillie limite et ne peut pas être retirée.

8. Mélangeur de médicaments selon la revendication 7, dans lequel la saillie limite est une mâchoire de serrage élastique dont les extrémités supérieures sont agencées uniformément et sont fixées le long de la périphérie de la paroi interne de la paroi de la tasse et dont les extrémités inférieures sont des extrémités libres, et la mâchoire de serrage élastique s'incline vers le centre de la tasse de mélange de médicaments à partir des extrémités supérieures fixes jusqu'aux extrémités inférieures libres ;

la distance de l'extrémité libre de la mâchoire de serrage élastique au fond de la tasse est sensiblement égale ou légèrement supérieure à l'épaisseur du bouchon de la bouteille de pénicilline, de sorte qu'après le montage de la bouteille de pénicilline sur le mélangeur de médicaments, le bouchon de la bouteille de pénicilline est serrée entre l'extrémité libre et le fond de la tasse par la mâchoire de serrage élastique.

9. Mélangeur de médicament selon la revendication 7, dans lequel la saillie limite est une base de serrage élastique qui comprend une bague de serrage qui est munie d'une mâchoire de serrage élastique sur la face inférieure, les extrémités supérieures de la mâchoire de serrage élastique sont agencées uniformément et sont fixées le long de la bague de serrage et les extrémités inférieures de la mâchoire de serrage élastique sont des extrémités libres, et la mâchoire de serrage élastique s'incline vers le centre de la tasse de mélange de médicaments des extrémités supérieures fixes aux extrémités inférieures libres ;

la distance de l'extrémité libre de la mâchoire de serrage élastique au fond de la tasse est sensiblement égale ou légèrement supérieure à l'épaisseur du bouchon de la bouteille de pénicilline, de sorte qu'après le montage de la bouteille de pénicilline sur le mélangeur de médicaments, le bouchon de la bouteille de pénicilline est serré entre l'extrémité libre et le fond de la tasse par la mâchoire de serrage élastique ;

la paroi interne de la paroi de la tasse est munie d'une structure limite et la base de serrage élastique est montée dans la tasse de mélange de médicaments et limitée par la structure limite.

10. Mélangeur de médicaments selon la revendication 7, dans lequel la base de serrage élastique a une plaque inférieure, une colonne de support, la bague de serrage et la mâchoire de serrage élastique qui sont intégrées, la plaque inférieure est munie d'une colonne de support à une périphérie pour supporter et fixer la bague de serrage ;

la plaque inférieure est pourvue d'un trou central à travers lequel l'aiguille de perforation pénètre de manière correspondante.

11. Mélangeur de médicaments selon l'une quelconque des revendications 1 à 10, dans lequel l'extrémité inférieure du passage de mélange de médicaments est munie d'un manche facile à casser pour sceller l'extrémité inférieure du passage de mélange de médicaments.

12. Mélangeur de médicaments avec une structure de renforcement, comprenant l'un quelconque des mélangeurs de médicaments selon les revendications 1 à 11 ;

la paroi de la tasse de mélange de médicaments est pourvue de nervures de renforcement, de manière à améliorer la résistance à la compression de la paroi de la tasse ;

les nervures de renforcement sont disposées intégralement au niveau d'un côté intérieur de la paroi de la tasse dans une direction de haut en bas et / ou dans une direction horizontale.

13. Deux ports rigides avec un mélangeur de médicaments, comprenant l'un quelconque des mélangeurs de médicaments selon les revendications 1 à 12, dans lesquels un passage de perfusion est agencé de l'autre côté de la base du mélangeur de médicaments par rapport au passage de mélange de médicaments.

14. Sac intraveineux souple à deux ports rigides, les deux ports rigides étant ceux de la revendication 13.

15. Sac intraveineux souple avec un mélangeur de médicaments, comprenant un mélangeur de médicaments et un sac intraveineux souple, le mélangeur de médicaments est celui selon l'une quelconque des revendications 1 à 12 ;

le mélangeur de médicaments est relié au sac souple par la base ;

comprenant en outre un orifice de perfusion qui est disposé sur le sac intraveineux souple du même côté du mélangeur de médicaments, ou disposé sur le sac intraveineux souple de l'autre côté opposé au mélangeur de médicaments.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**REFERENCES CITED IN THE DESCRIPTION**

**Patent documents cited in the description**

• EP 2845577 A